# EUROPEAN PATENT SPECIFICATION

(11) **EP 4 627 312 B1**
(45) Date of publication and mention of the grant of the patent: **04.11.2026**
(21) Application number: 23817475.9
(22) Date of filing: 17.11.2023
(51) Int. Cl.: G01N 1/04, G01N 1/08

(54) **A SAMPLING TOOL FOR SAMPLING REBURNED LIME FROM A LIME KILN, AND AN APPARATUS AND A METHOD FOR DETERMINING AMOUNT OF RESIDUAL CARBONATE IN REBURNED LIME SAMPLED FROM A LIME KILN**
PROBEWERKZEUG ZUR PROBEENTNAHME VON KALKRÜCKSTÄNDEN AUS EINEM KALKOFEN UND VORRICHTUNG UND VERFAHREN ZUR BESTIMMUNG DER RESTCARBONATMENGE IN KALKOFEN
OUTIL D'ÉCHANTILLONNAGE DE CHAUX RECALCINÉE DANS UN FOUR À CHAUX, ET APPAREIL ET PROCÉDÉ DE DÉTERMINATION DE QUANTITÉ DE CARBONATE RÉSIDUEL DANS LA CHAUX RECALCINÉE PRÉLEVÉE DANS UN FOUR À CHAUX

(30) Priority: 02.12.2022 FI 20226072
(43) Date of publication of application: 08.10.2025
(73) Proprietor: Andritz Oy, 00180 Helsinki (FI)
(72) Inventor: AALTONEN, Ossi, 15100 Lahti (FI); ANTIKAINEN, Airi, 00180 Helsinki (FI); MUSSALO, Mika, 48601 Kotka (FI); OKSMAN, Antti, 00180 Helsinki (FI); RÄSÄNEN, Jani, 50600 Mikkeli (FI); VEHVILÄINEN, Tuomo, 78201 Varkaus (FI)
(74) Representative: Devine, Christopher
(86) International application number: PCT/FI2023/050636
(87) International publication number: WO 2024/115809

(56) References cited:
- CN-U- 203 534 855
- CN-U- 208 313 621
- US-A- 4 702 114

## Description

### Field of the invention

The present invention relates to a sampling tool for sampling reburned lime from a lime kiln. Furthermore, the present invention relates to an apparatus and a method for determining amount of residual carbonate in reburned lime sampled from a lime kiln.

### Background of the invention

In a lime kiln lime mud (CaCO₃) is regenerated into reburned lime (CaO) with heat. Quality key performance indicator for burned lime in this process is the proportion of residual carbonate (CaCO₃ %) within the reburned lime.

Traditionally proportion of the residual carbonate is measured by taking samples manually from the burned lime at the lime kiln and by analyzing the samples in laboratory by using acid to determine amount of residual carbonate within the reburned lime.

A drawback of this known method is that it takes operators time during the production since they must carry out the sampling among their other tasks. It is also hazardous for the operators since reburned lime taken from the lime kiln is very hot (up to 1000 °C) as well as corrosive and irritating.

Chinese utility model publication CN203534855U discloses a sampling device for detecting pelletizing production process in combined iron and steel production. The sampling device of CN203534855U comprises a sampling shovel, an upper cover plate and an operating handle connected with the sampling shovel to operate the sampling device. The cover plate includes filter bores on cover plate and the sampling shovel bottom. The upper cover plate filter bore apertures are greater than the sampling shovel filter bores apertures. The size difference between the filter bore apertures depends on the particle size range of sampling.

US patent publication US4702114A discloses automatic device designed to collect precise volumes of various materials, including solids, liquids, and gases, from a specific area, even if that area is under different pressure or temperature conditions. It features a cylindrical probe that moves back and forth to gather samples, with a coaxial sleeve that helps seal and guide the probe during the process. The device includes a closed chamber for receiving and conditioning the samples, which can introduce liquids or gases to dissolve or suspend the materials for analysis. The system is designed to automatically introduce the probe and sleeve into the sampling area and then separate them once the sample is collected. After collecting a sample, the sleeve can be reintroduced to the sampling area to ensure the probe is sealed and ready for the next collection.

Chinese utility model publication CN208313621U discloses an automatic sampling device designed for a shellfish Rankine Bach annular sleeve kiln ash machine. It features a sleeve that covers the outer surface of the kiln's sample manipulator. The sample manipulator includes a stepping motor connected to a boom and a drive cylinder. A sampling shovel is attached to the end of the drive cylinder's telescopic rod for collecting samples. The device has a sealed door with a sampling window to ensure proper sampling without contamination. It includes a second bracket to support the drive cylinder when not in use. Both the first and second drive cylinders are electric, allowing for automated operation.

### Summary of the invention

The object of the present invention is to provide a sampling tool for sampling reburned lime sample from a lime kiln, and an apparatus and a method by means of which lime sampling, sample handling and analysis of the lime samples can be carried out quicker without manual work and by means of which exposure to hazards is reduced.

The above-described problem is solved by the present invention because the sampling tool according to the present invention has a preparation compartment which can be used for sampling the reburned lime sample from a lime kiln and screening of the sample when the sampling tool is within the lime kiln as well as for unloading the sample to further processing (e.g., to the grinding device for grinding the sample), and because these sample preparation phases can be carried out by using e.g., a remote controlled or automated sampling tool moving device remotely, thus avoiding the manual sampling and sample preparation work requiring operators working time and causing stress and safety risks. More specifically, the sampling tool according to the invention is characterized by what have been described in the independent claim 1. An apparatus for determining amount of residual carbonate in reburned lime sampled from a lime kiln is characterized by what have been described in the independent claim 7. A method for determining amount of residual carbonate in reburned lime sampled from a lime kiln is characterized by what have been described in the independent claim 10. The dependent claims 2 to 6 present some advantageous embodiments of the sampling tool according to the invention, the dependent claims 8 and 9 of the apparatus according to the invention and the dependent claim 11 of the method according to the invention.

An advantage of the present invention is that the sampling tool does not require tool change because all phases of the sampling can be carried out by using the same sampling tool, which accelerates the testing process and creates cost savings. Another advantage is that the sampling tool according to the present invention enables automatization of the testing by applying e.g., an industrial robot such that it provides high level of flexibility with respect to layout (compared to e.g., large conveyor systems). Further advantage is that the sampling tool of the present invention allows to do all the required sample handling tasks by using a single device which minimizes the required amount of separate equipment. This makes testing, among others, more straightforward and cost effective. Automatization and straightforwardness make sampling process quicker which allows application of higher analyzing frequency. Higher analyzing frequency enhances data resolution and enables the operators to control the lime reburning process more precisely. In addition, when applying the invention, due to automatization the operators are not required to carry out any of the sampling tasks manually, which relieve them to focus their primary work, which, among others, lowers the stress experienced due to the work. Also, because the robotized sampling is possible by using the sampling tool according to the invention, at least most of the above-described safety hazards relating to manual sampling can be eliminated.

### Brief description of the drawings

In the following, the invention will be described in more detail with reference to the appended drawings in which:
Fig. 1 shows an embodiment of a sampling tool according to the invention seen obliquely from above,
Fig. 2 shows a side view of the sampling tool shown in the figure 1 in a position wherein its analysis compartment is in upright position,
Fig. 3 shows a rear view of the sampling tool shown in the figure 1 and 2, and
Fig. 4 shows a front view of the sampling tool shown in the figures 1 to 3.

### Detailed description of some advantageous embodiments

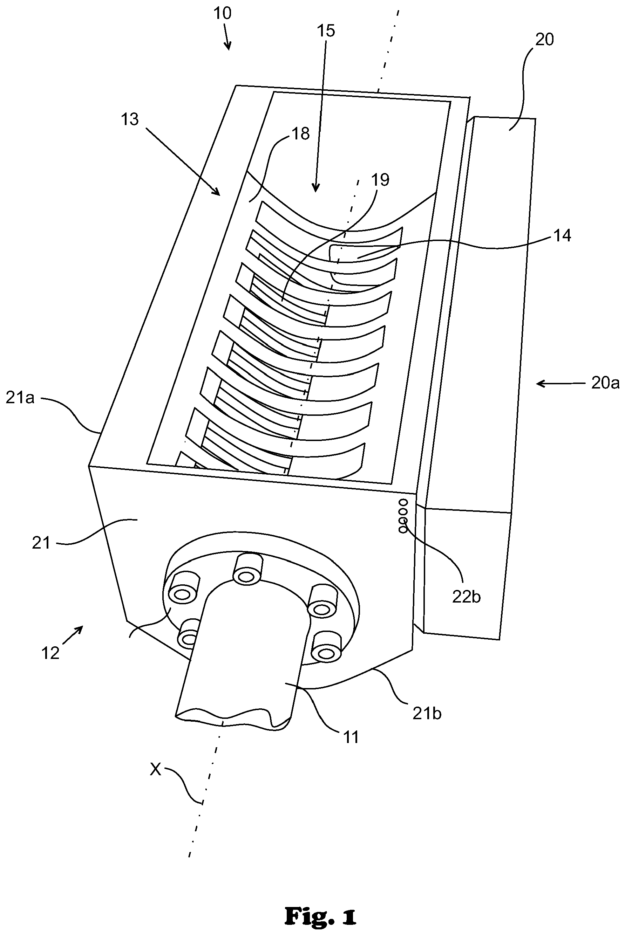

An embodiment of a sampling tool 10 shown in the figures 1-4 is suitable for sampling lime samples from several different positions of a lime kiln. Most typically the lime samples are taken from a chute of a lime kiln but sampling from other positions e.g., from channels and conveyors of the lime kiln, or from inside the lime kiln, is also possible. Therefore, when it is used hereinbelow generic definition that the sample *is taken or sampled from a lime kiln* it is meant that sample may be taken from any of these places.

The embodiment of the sampling tool 10 shown in the figures 1- 4 comprises holding device 11 for holding the sampling tool 10 in a lime kiln during the sampling. Holding device 11 for holding the sampling tool 10 may be e.g., elongated arm-like element which is preferably so long that it extends from outside the lime kiln to the position inside the lime kiln wherein the sampling takes place. The embodiment of figures 1-4 comprises further a preparation compartment 12 having sample inlet 13 for receiving a sample from the lime kiln, a sample outlet 14 for unloading the sample out from the preparation compartment 12 as well as a sieving arrangement 15 to limit the particle size range of the sample. In case of the embodiment of figures 1-4 the sieving arrangement 15 is capable to screen from the sample particles having size over a predetermined upper particle size limit Pₘₐₓ and a predetermined lower particle size limit Pₘᵢₙ. However, in some embodiments the sieving arrangement may be such that it is capable to screen from the sample only the particles having size over the predetermined upper particle size limit Pₘₐₓ (i.e., such that sample accepted by the sieving arrangement contains sample particles having particle size between 0 to Pₘₐₓ), or such that it is capable to screen from the sample only the particles having size under the predetermined lower particle size limit Pₘᵢₙ (i.e., such that sample accepted by the sieving arrangement contains sample particles having particle size between Pₘᵢₙ to size which still fits into the preparation compartment through the sample inlet).

In the embodiment shown in the figures 1-4 and wherein the sieving arrangement 15 is capable to screen from the sample particles having size over a predetermined upper particle size limit Pₘₐₓ and under the predetermined lower particle size limit Pₘᵢₙ the particle size is determined by the smallest diameter of the sieve opening of a first sieve 18 or a second sieve 19 of the sieving arrangement 15. More specifically, the diameter of the sieve opening defines the dimension of the sample particle which fits through the first sieve 18 or the second sieve 19. If the sieve opening has constant dimensions in all directions, it defines the largest sample particle that can pass the sieve. However, if the sieve openings dimensions vary (e.g., the sieve opening is a groove-like) then it defines the largest minimum dimension of the sample particle that may pass the sieve as the case is with the first sieve 18 and the second sieve 19 of the embodiment of figures 1-4. In case of the sampling tool shown in the figures 1-4 preferred value for Pₘₐₓ is 25 mm and for Pₘᵢₙ 5 mm. However, these values can vary in different embodiments of the invention. Furthermore, in such embodiments wherein the sieving arrangement is capable to screen from the sample particles having size over a predetermined upper particle size limit Pₘₐₓ, or under the predetermined lower particle size limit Pₘᵢₙ, there may be only one sieve arranged to screen from the sample the particles having the respective particle size.

In the embodiment of figures 1-4 the sample inlet 13 is an opening at the top of the preparation compartment 12, preferably being enough large and having shape that when the sampling tool 10 is placed, for instance under the chute of the lime kiln, lime can be received into the preparation compartment 12. In case of embodiment shown in the figures 1-4, sample inlet 13 is formed by open top side of the preparation compartment 12. In the other embodiments it may alternatively include only part of the top side. Furthermore, in some other embodiments the sample inlet may include additional element or elements and/or can be such that it allows taking the sample, e.g., from any suitable position inside the lime kiln instead of under the chute.

The sampling tool 10 shown in the figures 1 to 4 comprises also a cooling arrangement 16 arranged within the preparation compartment 12 to speed up the cooling of the sample in the preparation compartment 12. The lime sample taken from the lime kiln is very hot, typically it has temperature around 1000°C. Thus, it is important to cool the sample down after the sample has been screened in the lime kiln as quickly as possible to enable forthcoming phases, i.e., grinding and analysis of the sample without wasting time.

The first sieve 18 and the second sieve 19 have been arranged into the preparation compartment 12 such that the first sieve 18 rejects sample particles having size over a predetermined upper particle size limit Pₘₐₓ and a second sieve 19 sample particles having size under a predetermined lower particle size limit Pₘᵢₙ. The sample inlet 13 is above the first sieve 18 and the sample space 17 is a space between the first sieve 18 and the second sieve 19. Thus, when the screening is carried out the sample particles having size between the predetermined upper particle size limit Pₘₐₓ and lower particle size limit Pₘᵢₙ remains into the sample space 17.

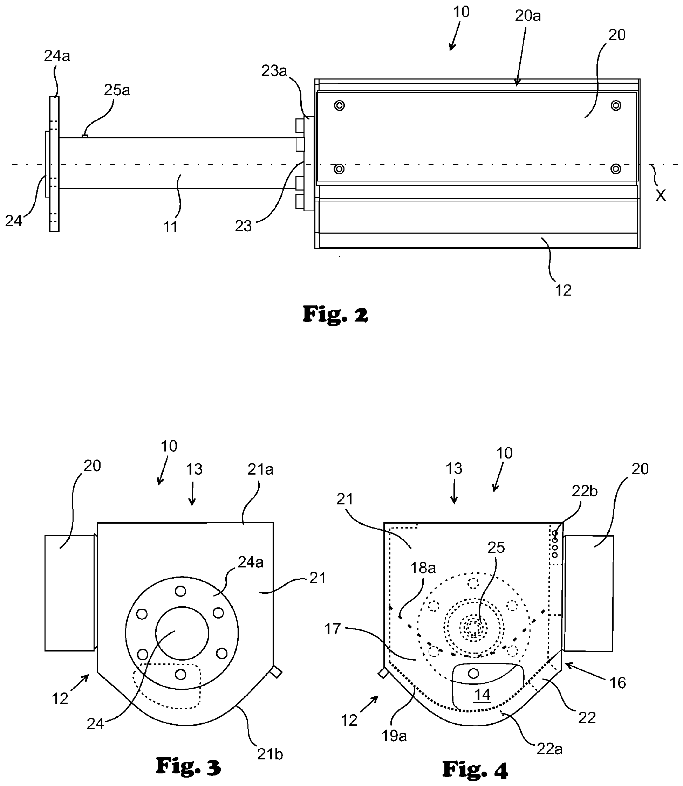

The sample outlet 14 is in the embodiment shown in the figures 1 to 4 an opening arranged to the preparation compartment 12 at the position of the sample space 17. More specifically, it is formed into the side wall 21 at the front end of the preparation compartment 12 as it can be seen from figure 4. Thus, the screened sample can be unloaded by tilting the sampling tool 10 in a slanted angle such a way that sample outlet 14 is directed downwards. In such embodiment which has only one sieve for screening the particles having size over a predetermined upper particle size limit Pₘₐₓ or under the lower particle size limit Pₘᵢₙ there may be only one sieve in the preparation compartment. In such case the only one sieve is located between the sample inlet and an impermeable bottom wall of the preparation compartment through which the sample particles accepted by the only one sieve cannot escape. Thus, in such embodiment the sample space is in within the sidewall between the only one sieve and the bottom wall. The sample outlet may be located similarly as it is in embodiment shown in the figures 1-4.

According to the invention, to accomodate the sample during its analysis the sampling tool 10 comprises an analysis compartment 20 being capable of receiving the sample from outside of the sampling tool 10. In the embodiment shown in figures 1-4 the analysis compartment 20 is a tray attached outside of preparation compartment 12 to the side wall 21 of the preparation compartment 12. Thus, it has only bottom wall and side wall and it is open from its top end to receive the sample to be analyzed. However, in some other embodiments the analysis compartment may not be part of the sampling tool but can be separate from it. Thus, it may be arranged e.g., in connection with a separate analysis device which is exchanged to the sampling tool moving device so that the separate analysis device replaces the part of the sampling tool containing the preparation compartment, and into which the prepared sample is loaded before analysis. Loading is most typically realized so that sample is received from the grinding device wherein the screened and cooled sample is unloaded from the preparation compartment after screening. Thus, in an embodiment of sampling tool which has not its own analysis compartment the analysis compartment is not present during the preparation phase, and in the other hand, the preparation compartment is not present during the analysis phase. Nevertheless, there may be also such embodiment where the analysis compartment is detachably attached to the sampling tool and thus in case of such embodiment the analysis of the sample can be carried out by using a sampling tool with the analysis compartment or by using separate device comprising an analysis compartment.

As can be seen from figures 1, 3 and 4, in the embodiment of figures 1-4 the analysis compartment 20 has been attached to the side wall 21 of the preparation compartment 12 in such orientation that its open end 20a opens perpendicularly to the direction in which the sample is received to the preparation compartment 12 through the sample inlet 13 (i.e., the sample to the analysis compartment is received perpendicularly to the receiving direction of the sample into the preparation compartment 12). This is advantageous since due to this feature previously analyzed sample is unloaded from the analysis compartment 20 at the same moment when the sampling tool 10 is turned in the position wherein the preparation compartment 12 is capable for receiving next sample to be prepared through the sample inlet 13. In other embodiments wherein the sampling tool includes an analysis compartment the angle between the open end of the analysis compartment and the said receiving direction of the sample into the preparation compartment may vary being some other than a perpendicular angle. Thus, it can be, for instance any angle, preferably between 30° to 120°, so that analyzed, previous sample is correspondingly unloaded when the sampling tool is turned in to the position in which preparation compartment 12 is ready for receiving the next sample to be prepared.

The preparation compartment 12 of the sampling tool 10 shown in the figures 1-4 comprises a side wall 21. Both, the first sieve 18 and the second sieve 19 comprise sieve plates 18a and 19a attached within the side wall at a distance from each other. The side wall 21 has upper edge 21a and lower edge 21b. As described above, in the embodiment of fig. 1-4 there is no top wall in the preparation compartment 12 whereas it is open from its top side. Thus, the top side of the preparation compartment 12 forms the sample inlet 13 for receiving the sample. The sieve plate 18a of the first sieve 18 is in between the upper edge 21a and lower edge 21b of the side wall 21 hence forming to the preparation compartment an *"intermediate floor".* The sieve plate 18a supports the sample when it is received into the preparation compartment 12 from the lime kiln through the sample inlet 13. The sieve plate 19a of the second sieve 19 is at the lower edge 21b of the side wall 21. However, in other embodiments like this, it may be also placed to some position between the sieve plate of the first sieve and the lower edge of the side wall such that appropriate space for sample particles passed through the first sieve is left between the sieve plate of the first sieve and sieve plate of the second sieve. The sieve plate 19a of the second sieve 19 forms in the sampling tool 10 the sole bottom wall to the preparation compartment 12. Thus, the particles of the sample having particle size below the predetermined lower particle size limit Pₘᵢₙ drop out from the preparation compartment 12 through the sieve plate 19b of the second sieve 19. In such embodiment wherein the mere sieve is a sieve for screening the particles having particle size under lower particle size limit Pₘᵢₙ the sieve may have only one sieve plate forming the bottom wall of the preparation compartment.

In the embodiment of the figures 1-4 the cooling arrangement 16 comprises a cooling medium channel 22 arranged within the preparation compartment 12. The cooling medium channel 22 is formed, in this embodiment, in the side wall of the preparation compartment by forming the side wall 21 double cover layers in between of which there is a hollow space as can be seen from figure 4. The hollow space is divided in sections which have been divided from each other by intermediate walls 22a that have been arranged within the side wall 21 such that cooling medium flows in all sections of the cooling medium channel 22. Thus, the cooling medium chills the whole side wall 21 when it flows through the cooling medium channel 22. Since, in this embodiment, the cooling medium is air, it is not necessary to return it back to its source whereas it can be released to atmosphere. This happens through the cooling medium outlets 22b shown in the figures 1 and 4. In the other embodiments the cooling medium can be some other fluid that is suitable for cooling the sampling tool after sampling. However, air is advantageous because any kind of equipment for storage and return it back to any storage is not required as the case is with the other cooling mediums. This makes cooling arrangement simpler and cheaper. Furthermore, in such system it is possible to utilize existing compressed air supply system such as the lime kiln's compressed air supply system.

In the embodiment of figures 1-4 the holding device 11 is an elongated arm-like element which has a first end 23 and a second end 24. In the second end there is a flange 24a by means of which the holding device can be attached to, or can be hold by a tool moving device, such as by an arm of an articulated robot. At the first end 23 there is a flange 23a by means of which the holding device 11 is secured to the front end of the preparation compartment 12. The holding device 11 is preferably designed so, that sampling tool 10 extends through a gate or hatch into the lime kiln such that tool moving device remains advantageously as much as possible outside the lime kiln. In this embodiment, the holding device comprises further a connecting conduit 25 connected to the cooling medium channel 22 of the preparation compartment 12 and a connector for connecting the connecting conduit 25 to a cooling medium supply device. Since the cooling medium is, in this case, air the connector can be e.g., pressure air connector. In the other embodiments cooling medium can be e.g., some liquid or other gas.

At least, in case when some liquid is used as a cooling medium, the system includes all the necessary equipment such as a storage container (e.g., a reservoir) for cooling liquid from which the cooling liquid is pumped to the sampling tool and into which the liquid is returned from the sampling tool after it has been circulated in the cooling channel of the sampling tool. In such case there could be also e.g., a heat exchanger or cooler for cooling the liquid before it is returned to the storage container.

When preparing the sampling tool according to figures 1 to 4 to the lime sampling the sampling tool 10 is hold from its holding device 11 or the holding device 11 is attached to a tool moving device from its flange 24a at its second end 24 fixedly. The tool moving device may be e.g., some suitable manipulator by means of which the sampling tool 10 can be moved into the lime kiln for taking a sample, and from the lime kiln to the other devices needed when carrying out the analysis. Preferably, the tool moving device is an articulated robot (having, e.g., six degrees of freedom) since by it the sampling tool can be moved most freely in desired positions and locations during the sampling phase and analysis phase. Articulated robot is also advantageous because it can be programmed for producing suitable screening movements to accomplish the screening e.g., in a lime drop chute inside the lime kiln. Also, the connector of the cooling arrangement and the cooling medium supply device may be prepared so that the sampling tool can be connected to the cooling medium supply device immediately after the sampling tool 10 is taken out from the lime kiln. Since the cooling medium used in embodiment of figures 1 to 4 is air, the cooling medium supply device is some suitable air supply device such as e.g., a compressed air supply system of the plant.

Sampling and determination of the amount of residual carbonate in a reburned lime sample by the sampling tool 10 according to figures 1 to 4 can be divided e.g., in following phases:
1. The sampling tool 10 is inserted in lime drop chute, or in other position in the lime kiln and sample is collected into the preparing compartment 12 of the sampling tool 10,
2. Particles of the lime over the predetermined upper size limit Pₘₐₓ are screened out by the first sieve 18. During screening, the sampling tool 10 is inside the chute and screening movement carried out by twisting the arm of the robot (or by respective part of the other tool moving device) back and forth causing the sampling tool 10 to turn correspondingly in respect of its longitudinal axis X. This results that reject (i.e., sample particles having size above the predetermined upper particle size limit Pₘₐₓ) drops out from preparation compartment 12. Accept, in this phase (i.e., sample particles having size under the predetermined upper particle size limit Pₘₐₓ) goes through sieve plate 18a of the first sieve 18 to the sample space 17.
3. Particles under the predetermined lower particle size limit Pₘᵢₙ are screened out by the sieve plate 19a of the second sieve 19. This happens to the sample particles that have passed the sieve plate 18a of the first sieve 18, i.e., which are in the sample space. Reject (i.e., sample particles having size under the predetermined lower particle size limit Pₘᵢₙ) formed in this phase of the screening goes through sieve plate 19a of the second sieve 19 and drops back into the lime kiln. Accept, (i.e., the sample particles having size between the upper particle size limit Pₘₐₓ and the lower particle size limit Pₘᵢₙ) remain inside the sample space 17.
4. The sampling tool 10 is moved to the cooling station were cooling air supply is connected to the holding device 11 which conducts it to the cooling channel 22 of the preparation compartment 12. Thus, the preparation compartment's 12 side wall 21 is cooled, and hence also the sample inside the sample space 17 cools down. As the cooling is carried out with the cooling arrangement 16 through the side wall 21 of the preparation compartment 12 and not e.g., by direct blowing, as the case is when applying presently known methods, the sample is not dusted to the atmosphere.
5. Cooled and screened sample is unloaded carefully from the sample space 17 through sample outlet 14 to a grinding device to perform grinding of the sample. The side wall 21 of the preparation compartment 12 at the position of the sample outlet 14 is shaped so, that in certain angle it keeps the sample in the sample space 17 while the sample is moved away from the position where the sampling was carried out. However, in this phase when the sampling tool 10 is tilted in slanted or vertical position the sample particles drop out from the sample space 17.
6. After grinding phase the sampling tool 12 is rotated by the tool moving device (i.e., e.g., by twisting the robot arm) such that open end of the analysis compartment 20 is directed upwards. In that position it can be used to collect the sample from the exit of the grinding device. Correct position is shown in the figure 2. Sample is then flattened by means a fixed scraper plate by moving the sampling tool in respect of the fixed scraper plate by the tool moving device (i.e., e.g., the robot arm) so that scarper readjusts the sample particles in the analysis compartment 20.
7. The final phase is to determine the amount of residual carbonate from the reburned lime by analyzing the prepared sample. In this phase the sample is taken to contact with analysis probe when the sample lies in the analysis compartment 20. The analysis may be carried out by e.g., using probe that determines the amount of residual carbonate by using Near Infrared Spectroscopy (NIRS). Alternatively, other types of probes being suitable for determining amount of residual carbonate may be applied. Furthermore, the analysis can be carried out by some other suitable methods. For instance, the traditional analysis method i.e., generally used calcinometer method to measure residual carbonate content (e.g., SCAN-32:98), or carbon content-based analysis method to measure residual carbonate content form reburned lime may be alternatively applied.

After the analysis phase the sampling cycle is started over. The phase 1 starts, and the used sample will be dropped into the drop chute when the sampling tool is twisted back into the position wherein it can receive the next lime sample from the chute into the preparation compartment 12. This way all the sample is taken back into the process and any waste is not formed.

In case of sampling tool having sieving arrangement which is such that it is capable to screen from the samples only the particles having size over a predetermined upper particle size limit Pₘₐₓ or particles having size under a predetermined lower particle size limit Pₘᵢₙ, sampling and determination of the amount of residual carbonate in a reburned lime sample is accomplished in corresponding manner as described above except that screening phase 2 or 3 is omitted respectively.

In an embodiment of apparatus for determining amount of residual carbonate in reburned lime sampled from a lime kiln, the apparatus comprises
- a sampling tool 10 of figures 1 to 4 for receiving a sample from the lime kiln, and for preparing the sample by at least screening the sample,
- an analysis compartment 20 at the sampling tool 10 as shown in the figures 1 to 4,
- a sampling tool moving device for moving the sampling tool 10 into the lime kiln, and from the lime kiln to a grinding device as well as for moving the sampling tool 10 with analysis compartment 20 of from the grinding device to the residual carbonate analysis device,
- grinding device for grinding the sample received from the preparation compartment 12 of the sampling tool 10, and
- residual carbonate analysis device for determining amount of residual carbonate from the sample in the analysis compartment 20.

In an embodiment of apparatus for determining amount of residual carbonate in reburned lime sampled from a lime kiln the sampling tool moving device is an articulated robot.

In an embodiment of apparatus for determining amount of residual carbonate in reburned lime sampled from a lime kiln the residual carbonate analysis device is a device determining amount of residual carbonate based on near infrared spectroscopy (NIRS).

In an embodiment of the method for determining amount of residual carbonate in reburned lime sampled from a lime kiln, the method comprises method steps of
- sampling a reburned lime sample from a lime kiln by using the sampling tool (10) according to any of claims 1 to 6,
- preparing the sample by screening the sample to limit the particle size range of the sample by using the sampling tool (10) of figures 1-4,
- unloading the sample from the preparation compartment 12 of the sampling tool 10 to a grinding device,
- grinding the sample by a grinding device,
- loading the sample to an analysis compartment 20 of the sampling tool 10 with the analysis compartment 20 of the sampling tool and determining the amount of residual carbonate from the sample in the analysis compartment.

In an embodiment of the method for determining amount of residual carbonate in reburned lime sampled from a lime kiln determination the amount of residual carbonate is carried out by applying near infrared spectroscopy (NIRS), by generally used calcinometer method to measure residual carbonate content (e.g., SCAN-32:98), or by a carbon content-based analysis method.

The sampling tool as well as the apparatus and the method for determining the amount of residual carbonate in reburned lime sample can be realized further in a way being different from the embodiments described above.

For instance, in some embodiments of the sampling tool the sieving arrangement may comprise a single sieve with adjustable sieve openings.

Such sieve may be placed into the preparation compartment, for instance similarly as the first sieve 18 in the embodiment of figures 1-4. Thus, by means of such sieving arrangement it is possible to screen from the sample particles having size over a predetermined upper particle size limit Pₘₐₓ and under the predetermined lower particle size limit Pₘᵢₙ by carrying out the screening in two separate phases. This would happen such a way that after the particles having size over a predetermined upper particle size limit Pₘₐₓ has been screened, in the first phase, the size of the sieve openings will be reduced to correspond particle size of the predetermined lower particle size limit Pₘᵢₙ, and then, in the second phase, the preparation compartment will be turned upside down to carry out the said second screening phase by using the sieve with the reduced sieve openings. Turning the preparation compartment upside down also unloads the reject of the first sieving phase (i.e., those too large particles) from above the sieve.

Thus, the invention is restricted to the embodiments described above but it can vary within the scope of the appended claims.

## Claims

1. A sampling tool (10) for sampling reburned lime from a lime kiln, comprising a holding device (11) for holding the sampling tool (10) in the lime kiln during the sampling, and a preparation compartment (12) having
- a sample inlet (13) for receiving a sample from the lime kiln into the preparation compartment (12),
- a sample outlet (14) for unloading the sample out from the preparation compartment (12),
- a sieving arrangement (15) for limiting the particle size range of the sample,
- a sample space (17) for the portion of the sample being screened by the sieving arrangement (15), wherein the sieving arrangement (15) comprises at least one of the following:
a first sieve (18) for screening from the sample the particles having size over an upper particle size limit Pₘₐₓ, a second sieve (19) for screening from the sample particles having particle size under a lower particle size limit Pₘᵢₙ,
and wherein, in the case where the sieving arrangement (15) comprises both the first sieve (18) for screening from the sample the particles having size over the upper particle size limit Pₘₐₓ and the second sieve (19) for screening from the sample particles having particle size under the lower particle size limit Pₘᵢₙ, the sample inlet (13) is above the first sieve (18), and the sample space (17) is a space between the first sieve (18) and the second sieve (19),
**characterized in that**
- the sampling tool (10) comprises an analysis compartment (20) being capable of receiving the sample from outside of the sampling tool (10) and to accommodate the sample during its analysis, and that
- the analysis compartment (20) is a tray being attached to the sampling tool (10) in a position wherein its open end (20a) is directed perpendicularly, or in a slanted angle in respect of the direction into which the preparation compartment (12) is arranged to receive the sample from the lime kiln such that when the sampling tool (10) is in said position to receive the sample from the lime kiln into the preparation compartment (12) the analysis compartment (20) is in position wherein it unloads it's content to unload the previous sample.

2. The sampling tool (10) according to claim 1, wherein the preparation compartment (12) comprises side wall (21) having upper edge (21a) and lower edge (21b), and wherein the first sieve (18) comprises a sieve plate (18a) and the second sieve (19) comprise sieve plate (19a), and wherein the sieve plates (18a, 19a) have been attached within the side wall (21) at a distance from each other.

3. The sampling tool (10) according to claim 2, wherein the sieve plate (18a) of the first sieve (18) is in between the upper edge (21a) and lower edge (21b) of the side wall (21), and wherein the sieve plate (19a) of the second sieve (19) is in between the sieve plate (18a) of the first sieve (18) and the lower edge (21b) of the side wall (21) or at the lower edge (21b) of the side wall (21).

4. The sampling tool (10) according to any of preceding claims, wherein the sampling tool (10) comprises a cooling arrangement (16) being capable to reduce the temperature of the sample in the preparation compartment (12).

5. The sampling tool (10) according to claim 4, wherein the cooling arrangement (16) comprises cooling medium circulation channel (22) arranged within the preparation compartment (12).

6. The sampling tool (10) according to any of preceding claims, wherein the holding device (11) comprises connecting conduit (25) connected to the cooling medium circulation channel (22) and a connector (25a) for connecting the connecting conduit (25) to a cooling medium supply device.

7. An apparatus for determining amount of residual carbonate in reburned lime sampled from a lime kiln comprising
- a sampling tool (10) according to any of claims 1 to 6 for receiving a sample from the lime kiln, and for preparing the sample by at least screening the sample,
- an analysis compartment being analysis compartment (20) as defined in claim 1,
- a sampling tool moving device for moving the sampling tool (10) into the lime kiln, and from the lime kiln to a grinding device as well as for moving the sampling tool (10) with analysis compartment (20) according to claim 1 from the grinding device to the residual carbonate analysis device,
- grinding device for grinding the sample received from the preparation compartment (12) of the sampling tool (10), and
- residual carbonate analysis device for determining amount of residual carbonate from the sample in the analysis compartment (20).

8. The apparatus according to claim 7, wherein the sampling tool moving device is an articulated robot.

9. The apparatus according to claim 7 or 8, wherein the residual carbonate analysis device is a device determining amount of residual carbonate based on near infrared spectroscopy (NIRS).

10. A method for determining amount of residual carbonate in reburned lime sampled from a lime kiln comprising method steps of
- sampling a reburned lime sample from a lime kiln by using the sampling tool (10) according to any of claims 1 to 6,
- preparing the sample by screening the sample to limit the particle size range of the sample by using the sampling tool (10) according to any of claims 1 to 6,
- unloading the sample from the preparation compartment (12) of the sampling tool (10) to a grinding device,
- grinding the sample by a grinding device,
- loading the sample to an analysis compartment (20) of the sampling tool (10) with the analysis compartment (20) according to claim 1 and determining the amount of residual carbonate from the sample in the analysis compartment.

11. The method according to claim 12, wherein determination the amount of residual carbonate is carried out by applying near infrared spectroscopy (NIRS), by generally used calcinometer method to measure residual carbonate content (e.g., SCAN-32:98), or by a carbon content-based analysis method.

## Patentansprüche

1. Probenahmewerkzeug (10) zur Probenahme von wieder gebranntem Kalk aus einem Kalkofen, das eine Haltevorrichtung (11) zum Halten des Probenahmewerkzeugs (10) im Kalkofen während der Probenahme und einen Präparationsraum (12) umfasst, der Folgendes aufweist
- einen Probeneinlass (13) zum Aufnehmen einer Probe aus dem Kalkofen in den Präparationsraum (12),
- einen Probenauslass (14) zum Entladen der Probe aus dem Präparationsraum (12),
- eine Siebanordnung (15) zum Begrenzen des Partikelgrößenbereichs der Probe,
- einen Probenraum (17) für den durch die Siebanordnung (15) gefilterten Teil der Probe, wobei die Siebanordnung (15) mindestens eines der Folgenden umfasst:
ein erstes Sieb (18) zum Filtern der Partikel mit einer Größe über einer Partikelgrößenobergrenze Pₘₐₓ aus der Probe, ein zweites Sieb (19) zum Filtern von Partikeln mit einer Partikelgröße unter einer Partikelgrößenuntergrenze Pₘᵢₙ aus der Probe,
und wobei, falls die Siebanordnung (15) sowohl das erste Sieb (18) zum Filtern der Partikel mit einer Größe über der Partikelgrößenobergrenze Pₘₐₓ aus der Probe als auch das zweite Sieb (19) zum Filtern von Partikeln mit einer Partikelgröße unter einer Partikelgrößenuntergrenze Pₘᵢₙ aus der Probe umfasst, der Probeneinlass (13) über dem ersten Sieb (18) liegt, und der Probenraum (17) ein Raum zwischen dem ersten Sieb (18) und dem zweiten Sieb (19) ist,
**dadurch gekennzeichnet, dass**
- das Probenahmewerkzeug (10) einen Analyseraum (20) umfasst, das in der Lage ist, die Probe von außerhalb des Probenahmewerkzeugs (10) aufzunehmen und die Probe während ihrer Analyse aufzunehmen, und dass
- der Analyseraum (20) eine Schale ist, die an dem Probenahmewerkzeug (10) in einer Position angebracht ist, in der ihr offenes Ende (20a) senkrecht oder in einem schrägen Winkel in Bezug auf die Richtung ausgerichtet ist, in der der Präparationsraum (12) zum Aufnehmen der Probe aus dem Kalkofen angeordnet ist, so dass, wenn sich das Probenahmewerkzeug (10) in der Position zum Aufnehmen der Probe aus dem Kalkofen in den Präparationsraum (12) befindet, sich der Analyseraum (20) in einer Position befindet, in der er seinen Inhalt zum Entladen der vorherigen Probe entlädt.

2. Probenahmewerkzeug (10) nach Anspruch 1, wobei der Präparationsraum (12) eine Seitenwand (21) mit einer Oberkante (21a) und einer Unterkante (21b) umfasst, und wobei das erste Sieb (18) eine Siebplatte (18a) umfasst und das zweite Sieb (19) eine Siebplatte (19a) umfasst, und wobei die Siebplatten (18a, 19a) innerhalb der Seitenwand (21) in einem Abstand voneinander angebracht wurden.

3. Probenahmewerkzeug (10) nach Anspruch 2, wobei sich die Siebplatte (18a) des ersten Siebs (18) zwischen der Oberkante (21a) und der Unterkante (21b) der Seitenwand (21) befindet, und wobei sich die Siebplatte (19a) des zweiten Siebs (19) zwischen der Siebplatte (18a) des ersten Siebs (18) und der Unterkante (21b) der Seitenwand (21) oder an der Unterkante (21b) der Seitenwand (21) befindet.

4. Probenahmewerkzeug (10) nach einem der vorhergehenden Ansprüche, wobei das Probenahmewerkzeug (10) eine Kühlanordnung (16) umfasst, die in der Lage ist, die Temperatur der Probe in dem Präparationsraum (12) zu reduzieren.

5. Probenahmewerkzeug (10) nach Anspruch 4, wobei die Kühlanordnung (16) einen Kühlmedienumwälzkanal (22) umfasst, der innerhalb des Präparationsraums (12) angeordnet ist.

6. Probenahmewerkzeug (10) nach einem der vorhergehenden Ansprüche, wobei die Haltevorrichtung (11) eine Verbindungsleitung (25), die mit dem Kühlmedienumwälzkanal (22) verbunden ist, und einen Verbinder (25a) zum Verbinden der Verbindungsleitung (25) mit einer Kühlmedienzufuhrvorrichtung umfasst.

7. Einrichtung zum Bestimmen einer Menge an Restcarbonat in wieder gebranntem Kalk, von dem eine Probe aus einem Kalkofen entnommen wird, umfassend
- ein Probenahmewerkzeug (10) nach einem der Ansprüche 1 bis 6 zum Aufnehmen einer Probe aus dem Kalkofen und zum Präparieren der Probe durch zumindest Filtern der Probe,
- einen Analyseraum, der der Analyseraum (20) nach Anspruch 1 ist,
- eine Probenahmewerkzeugbewegungsvorrichtung zum Bewegen des Probenahmewerkzeugs (10) in den Kalkofen und aus dem Kalkofen zu einer Mahlvorrichtung sowie zum Bewegen des Probenahmewerkzeugs (10) mit dem Analyseraum (20) nach Anspruch 1 aus der Mahlvorrichtung zu der Restcarbonatanalysevorrichtung,
- eine Mahlvorrichtung zum Mahlen der aus dem Präparationsraum (12) des Probenahmewerkzeugs (10) aufgenommenen Probe, und
- eine Restcarbonatanalysevorrichtung zum Bestimmen einer Menge an Restcarbonat aus der Probe in dem Analyseraum (20).

8. Einrichtung nach Anspruch 7, wobei die Probenahmewerkzeugbewegungsvorrichtung ein Gelenkroboter ist.

9. Einrichtung nach Anspruch 7 oder 8, wobei die Restcarbonatanalysevorrichtung eine Vorrichtung ist, die die Menge an Restcarbonat basierend auf Nahinfrarotspektroskopie (NIRS) bestimmt.

10. Verfahren zum Bestimmen einer Menge an Restcarbonat in wieder gebranntem Kalk, von dem eine Probe aus einem Kalkofen entnommen wird, umfassend die folgenden Verfahrensschritte
- Entnehmen einer Probe von wieder gebranntem Kalk aus einem Kalkofen unter Verwendung des Probenahmewerkzeugs (10) nach einem der Ansprüche 1 bis 6,
- Präparieren der Probe durch Filtern der Probe zum Begrenzen des Partikelgrößenbereichs der Probe unter Verwendung des Probenahmewerkzeugs (10) nach einem der Ansprüche 1 bis 6,
- Entladen der Probe aus dem Präparationsraum (12) des Probenahmewerkzeugs (10) zu einer Mahlvorrichtung,
- Mahlen der Probe durch eine Mahlvorrichtung,
- Beladen der Probe in einen Analyseraum (20) des Probenahmewerkzeugs (10) mit dem Analyseraum (20) nach Anspruch 1 und Bestimmen der Menge an Restcarbonat aus der Probe in dem Analyseraum.

11. Verfahren nach Anspruch 12, wobei das Bestimmen einer Menge an Restcarbonat durch Anwenden von Nahinfrarotspektroskopie (NIRS), durch ein üblicherweise verwendetes Calcinometerverfahren zum Messen eines Restcarbonatgehalts (z. B. SCAN-32:98) oder durch ein auf einem Kohlenstoffgehalt basierendes Analyseverfahren durchgeführt wird.

## Revendications

1. Outil d'échantillonnage (10) pour échantillonner de la chaux recalcinée provenant d'un four à chaux, comprenant un dispositif de maintien (11) pour maintenir l'outil d'échantillonnage (10) dans le four à chaux pendant l'échantillonnage, et un compartiment de préparation (12) présentant
- une entrée d'échantillon (13) pour recevoir un échantillon provenant du four à chaux dans le compartiment de préparation (12),
- une sortie d'échantillon (14) pour décharger l'échantillon du compartiment de préparation (12),
- un agencement de tamisage (15) pour limiter la gamme de tailles de particules de l'échantillon,
- un espace d'échantillon (17) pour la partie de l'échantillon qui est criblée par l'agencement de tamisage (15), dans lequel l'agencement de tamisage (15) comprend au moins l'un des éléments suivants :
un premier tamis (18) pour cribler à partir de l'échantillon les particules présentant une taille supérieure à une limite supérieure de taille de particule Pₘₐₓ, un deuxième tamis (19) pour cribler à partir de l'échantillon les particules présentant une taille de particule inférieure à une limite inférieure de taille de particule Pₘᵢₙ,
et dans lequel, dans le cas où l'agencement de tamisage (15) comprend à la fois le premier tamis (18) pour cribler à partir de l'échantillon les particules présentant une taille supérieure à la limite supérieure de taille de particule Pₘₐₓ et le deuxième tamis (19) pour cribler à partir de l'échantillon les particules présentant une taille de particule inférieure à la limite inférieure de taille de particule Pₘᵢₙ, l'entrée d'échantillon (13) est au-dessus du premier tamis (18), et l'espace d'échantillon (17) est un espace entre le premier tamis (18) et le deuxième tamis (19),
**caractérisé en ce que**
- l'outil d'échantillonnage (10) comprend un compartiment d'analyse (20) apte à recevoir l'échantillon depuis l'extérieur de l'outil d'échantillonnage (10) et à accueillir l'échantillon pendant son analyse, et **en ce que**
- le compartiment d'analyse (20) est un plateau qui est fixé à l'outil d'échantillonnage (10) dans une position dans laquelle son extrémité ouverte (20a) est dirigée perpendiculairement, ou suivant un angle incliné par rapport à la direction dans laquelle le compartiment de préparation (12) est agencé pour recevoir l'échantillon à partir du four à chaux de sorte que lorsque l'outil d'échantillonnage (10) est dans ladite position pour recevoir l'échantillon à partir du four à chaux dans le compartiment de préparation (12), le compartiment d'analyse (20) est dans une position dans laquelle il décharge son contenu pour décharger l'échantillon précédent.

2. Outil d'échantillonnage (10) selon la revendication 1, dans lequel le compartiment de préparation (12) comprend une paroi latérale (21) présentant un bord supérieur (21a) et un bord inférieur (21b), et dans lequel le premier tamis (18) comprend une plaque de tamis (18a) et le deuxième tamis (19) comprend une plaque de tamis (19a), et dans lequel les plaques de tamis (18a, 19a) ont été fixées à l'intérieur de la paroi latérale (21) à une distance l'une de l'autre.

3. Outil d'échantillonnage (10) selon la revendication 2, dans lequel la plaque de tamis (18a) du premier tamis (18) se trouve entre le bord supérieur (21a) et le bord inférieur (21b) de la paroi latérale (21), et dans lequel la plaque de tamis (19a) du deuxième tamis (19) se trouve entre la plaque de tamis (18a) du premier tamis (18) et le bord inférieur (21b) de la paroi latérale (21) ou au niveau du bord inférieur (21b) de la paroi latérale (21).

4. Outil d'échantillonnage (10) selon l'une quelconque des revendications précédentes, dans lequel l'outil d'échantillonnage (10) comprend un agencement de refroidissement (16) apte à réduire la température de l'échantillon dans le compartiment de préparation (12).

5. Outil d'échantillonnage (10) selon la revendication 4, dans lequel l'agencement de refroidissement (16) comprend un canal de circulation de fluide de refroidissement (22) agencé à l'intérieur du compartiment de préparation (12).

6. Outil d'échantillonnage (10) selon l'une quelconque des revendications précédentes, dans lequel le dispositif de maintien (11) comprend un conduit de raccordement (25) raccordé au canal de circulation de fluide de refroidissement (22) et un raccord (25a) permettant de raccorder le conduit de raccordement (25) à un dispositif d'alimentation en fluide de refroidissement.

7. Appareil permettant de déterminer la quantité de carbonate résiduel dans de la chaux recalcinée échantillonnée à partir d'un four à chaux comprenant
- un outil d'échantillonnage (10) selon l'une quelconque des revendications 1 à 6, permettant de recevoir un échantillon provenant du four à chaux, et permettant de préparer l'échantillon par au moins tamisage de l'échantillon,
- un compartiment d'analyse qui est un compartiment d'analyse (20) tel que défini dans la revendication 1,
- un dispositif de déplacement d'outil d'échantillonnage permettant de déplacer l'outil d'échantillonnage (10) dans le four à chaux, et à partir du four à chaux jusqu'à un dispositif de broyage ainsi que permettant de déplacer l'outil d'échantillonnage (10) avec le compartiment d'analyse (20) selon la revendication 1 à partir du dispositif de broyage jusqu'au dispositif d'analyse de carbonate résiduel,
- un dispositif de broyage permettant de broyer l'échantillon reçu à partir du compartiment de préparation (12) de l'outil d'échantillonnage (10), et
- un dispositif d'analyse de carbonate résiduel permettant de déterminer la quantité de carbonate résiduel de l'échantillon dans le compartiment d'analyse (20).

8. Appareil selon la revendication 7, dans lequel le dispositif de déplacement d'outil d'échantillonnage est un robot articulé.

9. Appareil selon la revendication 7 ou 8, dans lequel le dispositif d'analyse de carbonate résiduel est un dispositif déterminant la quantité de carbonate résiduel sur la base de la spectroscopie dans le proche infrarouge (SPIR).

10. Procédé permettant de déterminer la quantité de carbonate résiduel dans de la chaux recalcinée échantillonnée provenant d'un four à chaux, comprenant les étapes
- d'échantillonnage d'un échantillon de chaux recalcinée provenant d'un four à chaux en utilisant l'outil d'échantillonnage (10) selon l'une quelconque des revendications 1 à 6,
- de préparation de l'échantillon par tamisage de l'échantillon pour limiter la gamme de tailles de particules de l'échantillon en utilisant l'outil d'échantillonnage (10) selon l'une quelconque des revendications 1 à 6,
- de décharge de l'échantillon à partir du compartiment de préparation (12) de l'outil d'échantillonnage (10) jusqu'à un dispositif de broyage,
- de broyage de l'échantillon par un dispositif de broyage,
- de chargement de l'échantillon dans un compartiment d'analyse (20) de l'outil d'échantillonnage (10) avec le compartiment d'analyse (20) selon la revendication 1 et la détermination de la quantité de carbonate résiduel de l'échantillon dans le compartiment d'analyse.

11. Procédé selon la revendication 12, dans lequel la détermination de la quantité de carbonate résiduel est effectuée en appliquant une spectroscopie dans le proche infrarouge (SPIR), par un procédé calcimétrique généralement utilisé pour mesurer la teneur en carbonate résiduel (par exemple SCAN-32:98), ou par un procédé d'analyse basé sur la teneur en carbone.
